(19) Europäisches Patentamt / European Patent Office / Office européen des brevets

(11) EP 2 240 605 B1

(12) # EUROPEAN PATENT SPECIFICATION

(45) Date of publication and mention of the grant of the patent:
**06.08.2014 Bulletin 2014/32**

(21) Application number: **09702038.2**

(22) Date of filing: **16.01.2009**

(51) Int Cl.:
***C12Q 1/68*** *(2006.01)*

(86) International application number:
**PCT/FI2009/050037**

(87) International publication number:
**WO 2009/090311 (23.07.2009 Gazette 2009/30)**

(54) **NUCLEIC ACID PROBES AND BROAD-RANGE PRIMERS FOR DETECTING INFECTIOUS BACTERIA**

NUKLEINSÄURESONDEN UND BREITBAND-PRIMER ZUR DETEKTION VON INFEKTIÖSEN BAKTERIEN

SONDES D'ACIDES NUCLÉIQUES ET AMORCES À LARGE PORTÉE POUR LA DÉTECTION DES BACTÉRIES INFECTIEUSES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **17.01.2008 FI 20085039**
**12.02.2008 US 28057 P**

(43) Date of publication of application:
**20.10.2010 Bulletin 2010/42**

(60) Divisional application:
**13188069.2 / 2 716 770**

(73) Proprietor: **Mobidiag Oy**
**02150 Espoo (FI)**

(72) Inventors:
- **MÄKI, Minna-Mari**
  **00250 Helsinki (FI)**
- **PELLOSNIEMI, Jaakko**
  **00140 Helsinki (FI)**
- **PIIPARINEN, Pasi**
  **FI-02580 Siuntio (FI)**

(74) Representative: **Kolster Oy Ab**
**Iso Roobertinkatu 23**
**PO Box 148**
**00121 Helsinki (FI)**

(56) References cited:
**WO-A-00/61796 WO-A-2004/046379**

- **WATANABE K ET AL: "Design and evaluation of PCR primers to amplify bacterial 16S ribosomal DNA fragments used for community fingerprinting" JOURNAL OF MICROBIOLOGICAL METHODS, ELSEVIER, AMSTERDAM, NL, vol. 44, no. 3, 1 January 2001 (2001-01-01), pages 253-262, XP003016005 ISSN: 0167-7012**
- **HORNEK R ET AL: "Primers containing universal bases reduce multiple amoA gene specific DGGE band patterns when analysing the diversity of beta-ammonia oxidizers in the environment" JOURNAL OF MICROBIOLOGICAL METHODS, ELSEVIER, AMSTERDAM, NL, vol. 66, no. 1, 1 July 2006 (2006-07-01), pages 147-155, XP025073215 ISSN: 0167-7012 [retrieved on 2006-07-01]**
- **EHLERS B ET AL: "DETECTION OF NEW DNA POLYMERASE GENES OF KNOWN AND POTENTIALLY NOVEL HERPESVIRUSES BY PCR WITH DEGENERATE AND DEOXYINOSINE-SUBSTITUTED PRIMERS" VIRUS GENES, KLUWER ACADEMIC PUBLISHERS, BOSTON, US, vol. 18, no. 3, 1 January 1999 (1999-01-01), pages 211-220, XP008015647 ISSN: 0920-8569**
- **KILPATRICK DAVID R ET AL: "Serotype-specific identification of polioviruses by PCR using primers containing mixed-base or deoxyinosine residues at positions of codon degeneracy" JOURNAL OF CLINICAL MICROBIOLOGY, vol. 36, no. 2, February 1998 (1998-02), pages 352-357, XP002532261 ISSN: 0095-1137**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SANTOS SCOTT R ET AL: "Identification and phylogenetic sorting of bacterial lineages with universally conserved genes and proteins" ENVIRONMENTAL MICROBIOLOGY, BLACKWELL SCIENCE, OXFORD, GB, vol. 6, no. 7, 1 July 2004 (2004-07-01), pages 754-759, XP002447065 ISSN: 1462-2912**
- **KOSTIC ET AL: "A microbial diagnostic microarray technique for the sensitive detection and identification of pathogenic bacteria in a background of nonpathogens" ANALYTICAL BIOCHEMISTRY, ACADEMIC PRESS INC, NEW YORK, vol. 360, no. 2, 22 December 2006 (2006-12-22), pages 244-254, XP005762541 ISSN: 0003-2697**

SEQUENCE LISTING

<110> Mobidiag Oy

<120> Nucleic acid probes and broad-range primers

<130> 2062104PC

<160> 121

<170> PatentIn version 3.2

<210> 1
<211> 20
<212> DNA
<213> Artificial

<220>
<223> Primer

<220>
<221> misc_feature
<222> (3)..(3)
<223> n represents inosine

<220>
<221> misc_feature
<222> (6)..(6)
<223> n represents inosine

<220>
<221> misc_feature
<222> (9)..(9)
<223> n represents G or T

<220>
<221> misc_feature
<222> (15)..(15)
<223> n represents C or T

<220>
<221> misc_feature
<222> (18)..(18)
<223> n represents A, C or T

<400> 1
cgnccnggna tgtanatngg 20

<210> 2
<211> 23
<212> DNA
<213> Artificial

<220>
<223> Primer

<220>
<221> misc_feature
<222> (1)..(1)
<223> n represents A or G

<220>
<221> misc_feature
<222> (2)..(2)
<223> n represents A or C

<220>
<221> misc_feature
<222> (3)..(3)
<223> n represents inosine

<220>
<221> misc_feature
<222> (6)..(6) A or T
<223> n represents A or T

<220>
<221> misc_feature
<222> (9)..(9)
<223> n represents inosine

<220>
<221> misc_feature
<222> (12)..(12)
<223> n represents A or G

<220>
<221> misc_feature
<222> (15)..(15)
<223> n represents C or T

<220>
<221> misc_feature
<222> (18)..(18)
<223> n represents inosine

<220>
<221> misc_feature
<222> (21)..(21)
<223> n represents inosine

<400> 2
nnnccnacnc cntgnagncc ncc 23

<210> 3
<211> 23

<212> DNA
<213> Acinetobacter baumannii

<400> 3
gatgaagctt tagctggcca ctg 23

<210> 4
<211> 19
<212> DNA
<213> Acinetobacter baumannii

<400> 4
agtgtttcag ataatggcc 19

<210> 5
<211> 22
<212> DNA
<213> Acinetobacter baumannii

<400> 5
cagtgtttca gataatggcc gc 22

<210> 6
<211> 23
<212> DNA
<213> Acinetobacter baumannii

<400> 6
aattattgtc acgattcacg agg 23

<210> 7
<211> 23
<212> DNA
<213> Bacteroides fragilis group

<400> 7
agaaatctgc cctcgaagtt gcc 23

<210> 8
<211> 20
<212> DNA
<213> Bacteroides fragilis group

<400> 8
aatctgccct cgaagttgcc 20

<210> 9
<211> 23
<212> DNA
<213> Bacteroides fragilis group

<400> 9
gtattccggt agatttccac gaa 23

<210> 10
<211> 23
<212> DNA
<213> Bacteroides fragilis group

<400> 10
gaagacaact ctatcaccgt aca 23

<210> 11
<211> 22
<212> DNA
<213> Bacteroides fragilis group

<400> 11
gaccacatcg aagtgacaat ca 22

<210> 12
<211> 23
<212> DNA
<213> Bacteroides fragilis group

<400> 12
gaccacatcg aagtgacaat caa 23

<210> 13
<211> 25
<212> DNA
<213> Bacteroides fragilis group

<400> 13
acgaagataa ttcaatcacc gtaca 25

<210> 14
<211> 24
<212> DNA
<213> Bacteroides fragilis group

<400> 14
gaaatcagca ttggaagtag caat 24

<210> 15
<211> 23
<212> DNA
<213> Campylobacter jejuni

<400> 15
gatgtagaaa tcactactga agg 23

<210> 16
<211> 23
<212> DNA
<213> Campylobacter jejuni

<400> 16
tgtatagtta gtgataatgg tcg 23

<210> 17
<211> 24
<212> DNA
<213> Campylobacter jejuni

<400> 17
atatgccaac tttaactgtt gttt 24

<210> 18
<211> 23
<212> DNA
<213> Campylobacter jejuni

<400> 18
ttgatatgca cccaactgaa aat 23

<210> 19
<211> 20
<212> DNA
<213> Clostridium perfringens

<400> 19
gtagttgatg acggaagagg 20

<210> 20
<211> 20
<212> DNA
<213> Clostridium perfringens

<400> 20
gaagcattag ctggatactg 20

<210> 21
<211> 20
<212> DNA
<213> Clostridium perfringens

<400> 21
ggtagtacta gttcaagagg 20

<210> 22
<211> 20
<212> DNA
<213> Clostridium perfringens

<400> 22
gggaaaacca actgtagaag 20

<210> 23
<211> 23
<212> DNA
<213> Enterobacter aerogenes

<400> 23
acatcgttgt tacgatccac agc 23

<210> 24
<211> 23
<212> DNA
<213> Enterobacter aerogenes / Klebsiella pneumoniae

<400> 24
ccgcggcatc ccaaccggca ttc 23

<210> 25
<211> 19

<212> DNA
<213> Enterobacter aerogenes / Klebsiella pneumoniae

<400> 25
tctccgcggc ggaagttat 19

<210> 26
<211> 21
<212> DNA
<213> Enterobacter cloacae

<400> 26
gatccatgcg gacaactccg t 21

<210> 27
<211> 21
<212> DNA
<213> Enterobacter cloacae

<400> 27
ggtcacgatc catgcggaca a 21

<210> 28
<211> 19
<212> DNA
<213> Enterobacter cloacae

<400> 28
agagggcgta tctgctgcg 19

<210> 29
<211> 18
<212> DNA
<213> Enterobacteriaceae family

<400> 29
ggatgacggc accggtct 18

<210> 30
<211> 19
<212> DNA
<213> Enterobacteriaceae family

<400> 30
ctatcgacga agcgctcgc 19

<210> 31
<211> 19
<212> DNA
<213> Enterobacteriaceae family

<400> 31
tatcgacgaa gccctcgcg 19

<210> 32
<211> 20
<212> DNA
<213> Enterobacteriaceae family

<400> 32
atcgacgaag ccctcgcggg 20

<210> 33
<211> 23
<212> DNA
<213> Enterococcus faecalis

<400> 33
agctgtggaa acagtgttta cag 23

<210> 34
<211> 23
<212> DNA
<213> Enterococcus faecium

<400> 34
tatcaccgtt attgacgacg gtc 23

<210> 35
<211> 23
<212> DNA
<213> Enterococcus faecium

<400> 35
tatccaggca aaaactggac gtc 23

<210> 36
<211> 23
<212> DNA
<213> Enterococcus faecium

<400> 36
ggatacctgt agatatccag gca 23

<210> 37
<211> 21
<212> DNA
<213> Enterococcus faecium

<400> 37
tcgatcgatg aagtattagc a 21

<210> 38
<211> 23
<212> DNA
<213> Enterococcus faecium

<400> 38
actagttctg agggtctgca tca 23

<210> 39
<211> 23
<212> DNA
<213> Escherichia coli

<400> 39
aaattatcgt caccattcac gcc 23

<210> 40
<211> 21
<212> DNA
<213> Escherichia coli

<400> 40
ggcgcggcat tccgaccggt a 21

<210> 41
<211> 22
<212> DNA
<213> Escherichia coli

<400> 41
cgccgataac tctgtctctg ta 22

<210> 42
<211> 19
<212> DNA
<213> Escherichia coli

<400> 42
tatcggcggc ggaagtgat 19

<210> 43
<211> 19
<212> DNA
<213> Escherichia coli

<400> 43
acgggcgcgg cattccgac 19

<210> 44
<211> 18
<212> DNA
<213> Escherichia coli

<400> 44
aagagggcgt atcggcgg 18

<210> 45
<211> 23
<212> DNA
<213> Haemophilus influenzae

<400> 45
tctgtatcgg tgcaagatga tgg 23

<210> 46
<211> 22
<212> DNA
<213> Haemophilus influenzae

<400> 46
ggccattgtt ccgatattat cg 22

<210> 47
<211> 21

<212> DNA
<213> Haemophilus influenzae

<400> 47
gttccgatat tatcgtgaca a 21

<210> 48
<211> 20
<212> DNA
<213> Haemophilus influenzae

<400> 48
ctgtggatat tcatcctgaa 20

<210> 49
<211> 22
<212> DNA
<213> Haemophilus influenzae

<400> 49
atgccattga tgaagccctc gc 22

<210> 50
<211> 24
<212> DNA
<213> Enterobacter aerogenes / Klebsiella oxytoca

<400> 50
ttactgtaaa gacatcgttg ttac 24

<210> 51
<211> 23
<212> DNA
<213> Enterobacter aerogenes Klebsiella oxytoca

<400> 51
actgtaaaga catcgttgtt acg 23

<210> 52
<211> 22
<212> DNA
<213> Klebsiella oxytoca

<400> 52
gcggtattcc aaccggtatt ca 22

<210> 53
<211> 21
<212> DNA
<213> Klebsiella oxytoca

<400> 53
ggccgcggta ttccaaccgg t 21

<210> 54
<211> 22
<212> DNA
<213> Klebsiella pneumoniae

<400> 54
tactgcaaag atatcgttgt ca 22

<210> 55
<211> 21
<212> DNA
<213> Klebsiella pneumoniae

<400> 55
ctgcaaagat atcgttgtca c 21

<210> 56
<211> 23
<212> DNA
<213> Listeria monocytogenes

<400> 56
tacaatcgaa gctgataaca gca 23

<210> 57
<211> 23
<212> DNA
<213> Listeria monocytogenes

<400> 57
actgttcgtg ataacggacg tgg 23

<210> 58
<211> 20
<212> DNA
<213> Listeria monocytogenes

<400> 58
ggtcgtccaa cagtagaagt 20

<210> 59
<211> 21
<212> DNA
<213> Listeria monocytogenes

<400> 59
gcaattgatg aagcacttgc t 21

<210> 60
<211> 21
<212> DNA
<213> Neisseria meningitidis

<400> 60
aatcacggta acgatacacg c 21

<210> 61
<211> 20
<212> DNA
<213> Neisseria meningitidis

<400> 61
ccattgacga agcactcgcc 20

<210> 62
<211> 20
<212> DNA
<213> Neisseria meningitidis

<400> 62
gtattggaca acgccattga 20

<210> 63
<211> 21
<212> DNA
<213> Neisseria meningitidis

<400> 63
ggacattgcg acaaaatcac g 21

<210> 64
<211> 26
<212> DNA
<213> Proteus genus

<400> 64
attgtcacta tccattcaga taactc 26

<210> 65
<211> 21
<212> DNA
<213> Proteus genus

<400> 65
catggtgtag gggtttctgt t 21

<210> 66
<211> 22
<212> DNA
<213> Proteus mirabilis

<400> 66
acgggtattc atgaagaaga gg 22

<210> 67
<211> 23
<212> DNA
<213> Proteus mirabilis

<400> 67
gtattccaac gggtattcat gaa 23

<210> 68
<211> 21
<212> DNA
<213> Proteus mirabilis

<400> 68
atgaagaaga gggcgtttct 21

<210> 69
<211> 21

<212> DNA
<213> Proteus vulgaris

<400> 69
accaagaaga gggtgtttca g 21

<210> 70
<211> 21
<212> DNA
<213> Proteus vulgaris

<400> 70
accaagaaga gggtgtttca g 21

<210> 71
<211> 21
<212> DNA
<213> Pseudomonas aeruginosa

<400> 71
gatatccaca aggaagaagg g 21

<210> 72
<211> 21
<212> DNA
<213> Pseudomonas aeruginosa

<400> 72
tcactgtccg cgacaatgga c 21

<210> 73
<211> 22
<212> DNA
<213> Pseudomonas aeruginosa

<400> 73
gccggttayt gcagcgaaat ca 22

<210> 74
<211> 20
<212> DNA
<213> Salmonella enterica subspecies enterica

<400> 74
tcgtcgtgac tattcacgcc 20

<210> 75
<211> 23
<212> DNA
<213> Salmonella enterica subspecies enterica

<400> 75
taacggatga tggccgtggc att 23

<210> 76
<211> 20
<212> DNA
<213> Serratia marcescens

<400> 76
ttcagccgca gaggtcatca 20

<210> 77
<211> 21
<212> DNA
<213> Serratia marcescens

<400> 77
attcaggtca ccatccatgc c 21

<210> 78
<211> 20
<212> DNA
<213> Serratia marcescens

<400> 78
tcaggtcacc atccatgccg 20

<210> 79
<211> 19
<212> DNA
<213> Serratia marcescens

<400> 79
cgacaactcg gtatcggtg 19

<210> 80
<211> 25
<212> DNA
<213> Staphylococcus aureus

<400> 80
aatagtatcg atgaagcatt agctg 25

<210> 81
<211> 20
<212> DNA
<213> Staphylococcus aureus

<400> 81
aacggacgtg gtatcccagt 20

<210> 82
<211> 23
<212> DNA
<213> Staphylococcus aureus

<400> 82
ggacgtggta tcccagttga tat 23

<210> 83
<211> 23
<212> DNA
<213> Staphylococcus aureus

<400> 83
tccagctgtc gaagttattt taa 23

<210> 84
<211> 25
<212> DNA
<213> Staphylococcus epidermidis

<220>
<221> misc_feature
<222> (17)..(17)
<223> n represents A or G

<400> 84
tagtcatatt gaagttntaa ttgag 25

<210> 85
<211> 20
<212> DNA
<213> Staphylococcus epidermidis

<400> 85
gccctgctgt cgaagttatc 20

<210> 86
<211> 24
<212> DNA
<213> Staphylococcus epidermidis

<400> 86
cattagcagg ttatgctagt cata 24

<210> 87
<211> 20
<212> DNA
<213> Staphylococcus epidermidis

<400> 87
acgccctgct gtcgaagtta 20

<210> 88
<211> 25
<212> DNA
<213> Staphylococcus epidermidis

<400> 88
aaagatggga cgccctgctg tcgaa 25

<210> 89
<211> 23
<212> DNA
<213> Staphylococcus genus

<400> 89
ggcattcctg ttgatattca aga 23

<210> 90
<211> 23
<212> DNA
<213> Staphylococcus genus

<400> 90
tcaacttcag aaaaaggttt aca 23

<210> 91
<211> 23
<212> DNA
<213> Staphylococcus genus

<400> 91
tcaacttcag aaagaggttt gca 23

<210> 92
<211> 23
<212> DNA
<213> Staphylococcus genus

<400> 92
gttgataata gtattgacga agc 23

<210> 93
<211> 22
<212> DNA
<213> Staphylococcus genus

<400> 93
cgcccagcag ttgaagttat ct 22

<210> 94
<211> 23
<212> DNA
<213> Staphylococcus genus

<400> 94
ccagcagttg aagttatctt aac 23

<210> 95
<211> 22
<212> DNA
<213> Staphylococcus genus

<400> 95
ccagcagttg aggttatttt aa 22

<210> 96
<211> 19
<212> DNA
<213> Staphylococcus genus

<400> 96
ggggcgccca gcagttgaa 19

<210> 97
<211> 17
<212> DNA
<213> Stenotrophomonas maltophilia

<400> 97
tcggtgtccg acaacgg 17

<210> 98
<211> 20
<212> DNA
<213> Stenotrophomonas maltophilia

<400> 98
ggcaagcacg agcagatgag 20

<210> 99
<211> 20
<212> DNA
<213> Stenotrophomonas maltophilia

<400> 99
gccgaagtgg tgatgacggt 20

<210> 100
<211> 23
<212> DNA
<213> Streptococcus agalactiae

<400> 100
ttacattgaa ccagataact cta 23

<210> 101
<211> 23
<212> DNA
<213> streptococcus agalactiae

<400> 101
tggaagacca gctgtagaga cag 23

<210> 102
<211> 22
<212> DNA
<213> streptococcus agalactiae

<400> 102
ccttagctgg atttgcagga ca 22

<210> 103
<211> 23
<212> DNA
<213> Streptococcus agalactiae

<400> 103
attccggtag atatccaaga aaa 23

<210> 104
<211> 23
<212> DNA
<213> streptococcus dysgalactiae

<400> 104
atcgtcgaca attctattga cga 23

<210> 105
<211> 22

<212> DNA
<213> Streptococcus dysgalactiae

<400> 105
attagccgga tttgctactc ac 22

<210> 106
<211> 22
<212> DNA
<213> Streptococcus dysgalactiae

<400> 106
aggcagataa ttcaatcaca gt 22

<210> 107
<211> 22
<212> DNA
<213> Streptococcus dysgalactiae

<400> 107
ctactcacat ccaggtgttt at 22

<210> 108
<211> 20
<212> DNA
<213> Streptococcus dysgalactiae

<400> 108
atccaggtgt ttatcgaggc 20

<210> 109
<211> 21
<212> DNA
<213> Streptococcus pneumoniae

<400> 109
tcctgctgtt gagaccgtct t 21

<210> 110
<211> 24
<212> DNA
<213> Streptococcus pneumoniae

<400> 110
tcaacctcaa aagaaggtct tcac 24

<210> 111
<211> 22
<212> DNA
<213> Streptococcus pneumoniae

<400> 111
agtttttatt gagccagatg at 22

<210> 112
<211> 20
<212> DNA
<213> Streptococcus pyogenes

<400> 112
gtcccgccgt tgaaacagtt 20

<210> 113
<211> 23
<212> DNA
<213> Streptococcus pyogenes

<400> 113
tttttacagt cttacacgca ggt 23

<210> 114
<211> 29
<212> DNA
<213> Streptococcus pyogenes

<400> 114
gcaggttttg cctctcatat taaagtctt 29

<210> 115
<211> 22
<212> DNA
<213> Streptococcus pyogenes

<400> 115
ttacagtctt acacgcaggt gg 22

<210> 116
<211> 23
<212> DNA
<213> Enterococcus faecalis

<400> 116
ctggtatccc aacagtagaa gta 23

<210> 117
<211> 23
<212> DNA
<213> Enterococcus faecium

<400> 117
tttggtcgag gagggtacaa aac 23

<210> 118
<211> 23
<212> DNA
<213> Staphylococcus aureus

<400> 118
gtaaaccgac agtcgaagtt atc 23

<210> 119
<211> 20
<212> DNA
<213> Staphylococcus aureus

<400> 119
cgtggtatgc caacaggtat 20

<210> 120
<211> 24
<212> DNA
<213> Staphylococcus aureus

<400> 120
gtatttctat agaagataat ggac 24

<210> 121
<211> 24
<212> DNA
<213> Staphylococcus epidermidis

<400> 121
tggttatggt gatgcgatta cagt 24

**Description**

FIELD OF THE INVENTION

The invention relates to improved broad-range primers that are useful in the identification of bacterial species and in the diagnosis of bacterial infections. The invention also relates to a method of detecting and identifying infection causing bacteria in a sample.

BACKGROUND OF THE INVENTION

Sepsis is a severe life-threatening systemic disease resulting from an infection of the bloodstream by toxin-producing bacteria. In sepsis, dysfunction of the circulatory system may lead to multiple organ failures and to a drop in the blood pressure, resulting in shock. Worldwide, sepsis is the most common cause of death in intensive care units with a mortality rate ranging from 20% to 60%.

Diagnosis of sepsis is mainly based on bacterial culture testing. Bacterial culture testing is, however, relatively slow, and diagnostic methods based on cultivation usually give results only days, sometimes up to weeks after sampling. Furthermore, cultivation of bacteria is not always successful under laboratory conditions. This can be a consequence of either the fact that the culture method used is not applicable for the bacterial species in question, or the fact that the patient has received antimicrobial therapy before the sample is taken. Molecular methods based on amplification and hybridization of nucleic acids aim to solve the above-mentioned problems of bacterial culture testing. In such methods, the pathogen is simultaneously detected and identified, resulting in more rapid diagnostics and obviating the need for additional culture tests.

One molecular method used in bacterial diagnostics is the so-called broad-range PCR (polymerase chain reaction) based on the use of broad-range primers. The most common broad-range PCR methods use primers that recognize conserved DNA sequences of bacterial chromosomal genes encoding ribosomal RNA (16S rDNA/rRNA or 23S rDNA/rRNA). In bacterial identification based on broad-range PCR, the actual identification step is carried out by cloning and sequencing the amplified PCR product (e.g., EP-B1 613 502, US patent publications 6,001,564, and 2002/0055101).

The broad-range bacterial PCR method has been to some extent applied to clinical bacterial diagnostics, although it is best suited for the identification of bacterial and fungal species from culture isolates. For this purpose commercial tests, such as MicroSeq® (Applied Biosystems), have been developed. These tests, however, are not widely used because sequencing of PCR products is time-consuming and labor-intensive. The assays themselves and the necessary equipment, such as sequencing instruments, are expensive, and performing the tests requires specially trained personnel. Furthermore, in polymicrobial infections extensive cloning experiments may be needed.

The use of ribosomal RNA also includes some drawbacks. Distinguishing the related bacterial species from each other with the help of rRNA molecules is difficult, because the sequences of these molecules do not contain enough variable regions when they are compared with each other. Even if differences between various bacterial species may be found, these variable sites are generally divided across the whole rRNA molecule (e.g., the length of 16S rRNA is about 1500 nucleotides). This limits the use of molecular methods for diagnostics. In practice, the related bacterial species can thus be distinguished from each other only by sequencing the whole rRNA encoding gene. Even this approach is not always sufficient for bacterial identification. In phylogenetic analysis, *gyrB* gene has reported to be more discriminative in bacterial identification than for example 16S rRNA gene (Dauga, Int. J. Syst. Evol. Microbiol., 52: 531-547, 2002).

International patent publication WO2004046379 discloses tools and methods for diagnosis of bacterial infections, especially respiratory tract infections. This publication discloses broad-range primers that originate from conserved regions of genes encoding topoisomerases, especially the *gyrB* and *parE* genes, and bacterial species-specific oligonucleotide probes that originate from hyper-variable regions situated between the conserved regions. Using these broad-range primers and bacterial species-specific probes, single or even multiple infection-causing bacteria can be simultaneously detected and identified. The probes, however, cover only certain bacterial species. Furthermore, they do not allow identification of all bacterial strains due to variation occurring in their DNA sequences. Some of the probes also cross-react to some extent with untargeted clinically relevant pathogens leading to potential misidentification. In addition, the probes may possess low hybridization efficiency. In regard to the primers, they are highly degenerated demanding a complicated oligosynthesis with possible batch-to-batch variation. Also, the concentration of a single oligo variant in a PCR reaction mixture may be low and thus, the amplification of some strains could be inefficient. Thus, there is a real need in the art for improved tools and methods for bacterial diagnostics.

BRIEF DESCRIPTION OF THE INVENTION

The purpose of the present invention is to provide improved tools and methods, which are useful in bacterial diagnostics of infectious diseases, especially sepsis, but which lack the drawbacks of bacterial diagnostics described above. In particular, the purpose of the invention is to provide novel tools and methods, which are useful in detecting and identifying bacteria based on molecular methods, the tools and methods being sensitive, effective, and species or higher-level taxa specific, and being capable of identifying selectively the desired bacteria. A further purpose of the invention is to provide methods, by which it is possible to detect and identify simultaneously multiple infectious bacteria, especially those that cause sepsis, more accurately than previously possible, whereby for instance appropriate and effective antimicrobial therapy can be prescribed to the patient at an earlier stage of the infection so that the duration of the infection becomes shorter and the risk of potentially harmful, even life-threatening complications is reduced.

The present invention relates to a method of detecting and identifying infection-causing bacteria in a sample, which method comprises the steps of

a) amplifying DNA from said sample using a mixture of DNA primers that comprises sequence<u>s which</u> hybridize with the conserved regions of genes encoding topoisomerases, <u>said mixture comprising</u> SEQ ID NOs: 1 and 2,
b) contacting the amplified DNA with at least one oligonucleotide probe which hybridizes with a bacterial species or higher-level taxon specific sequence of a hyper-variable region that is situated between the regions with which said DNA primers hybridize, comprising a sequence selected from a group consisting of SEQ ID NOs: 3 to 121 and complementary sequences thereof, and
c) detecting any hybridization complexes formed and identifying said infection-causing bacteria based on said detection of said hybridization complex.

In one preferred embodiment of the method according to the present invention, the sample to be analyzed is a clinical sample.

In another preferred embodiment, the infection-causing bacteria are bacteria that cause sepsis. Examples of such bacteria belong taxonomically to Bacillales, Bacteroidetes, Clostridia, Lactobacillales, and Proteobacteria (Beta, Epsilon, Gamma). More specifically, the bacteria to be detected and identified are selected from the Proteobacteria, specifically Betaproteobacteria including *Neisseria meningitidis,* Epsilonproteobacteria including *Campylobacter jejuni,* and Gammaproteobacteria including *Acinetobacter baumannii, Enterobacter aerogenes, Enterobacter cloacae, Escherichia coli, Haemophilus influenzae, Klebsiella oxytoca, Klebsiella pneumoniae, Proteus mirabilis, Proteus vulgaris, Pseudomonas aeruginosa, Salmonella enterica* subspecies *enterica, Serratia marcescens, and Stenotrophomonas maltophilia,* the Lactobacillales including *Enterococcus faecalis, Enterococcus faecium, Streptococcus agalactiae, Streptococcus dysgalactiae, Streptococcus pneumoniae,* and *Streptococcus pyogenes,* the Bacillales including *Listeria monocytogenes, Staphylococcus aureus, Staphylococcus epidermidis,* Bacteroidetes including *Bacteroides fragilis,* and Clostridia including *Clostridium perfringens.* In one embodiment, *Staphylococcus* genus, *Proteus* genus and/or *Enterobacteriaceae* family members are detected and identified.

Furthermore, one preferred embodiment of the method of the invention comprises amplification the DNA from a biological specimen by PCR. In another preferred embodiment of the method of the invention, a suitably labeled nucleotide or a labeled primer is used in the amplification of DNA from a biological specimen in order to generate a detectable target strand.

In still another preferred embodiment of the method of the invention, the amplified and possibly labeled target DNA is contacted with at least one herein-disclosed species or higher-level taxa specific oligonucleotide probe selected from a group consisting of SEQ ID NOs: 3 to 121 and complementary sequences thereof placed on a suitable solid support, such as treated glass surface or silicon based biochip, or in a solution. In a still further embodiment, microarray technology is used.

The present method may consist of the steps described above or it may comprise for example DNA amplification with further primers and/or contacting the amplified DNA with further probes. In some embodiments, the method may also comprise further steps, such as washing and incubation steps.

The present invention further relates to a DNA primer <u>mixture</u> that comprise<u>s</u> sequences that hybridize with conserved regions of genes encoding topoisomerases of bacteria, said mixture comprising SEQ ID NOs: 1 and 2. The present invention also relates to the use of the above-mentioned improved primers in the amplification of topoisomerase genes, especially genes encoding the gyrB and the parE proteins.

The present invention further relates to a kit for use in the detection and identification of infection-causing bacteria, especially those causing sepsis, comprising

a) at least one DNA primer according to the present invention,
b) at least one bacterial species or higher-level taxa specific oligonucleotide probe disclosed herein, optionally attached on a solid support,
c) optionally, at least one positive and/or negative control probe sequence, and
d) optionally, reagents required in the amplification, purification, hybridization, washing, and/or detection steps.

In one embodiment, the kit consists of the components described above or it may comprise further primers and/or probes, as well as other appropriate reagents.
In further embodiment, the kit comprises probes specific for one or more taxonomical groups or bacterial species.

BRIEF DESCRIPTION OF THE DRAWINGS

Figure 1 shows the result of the PCR reaction carried out using the primer pair mixture disclosed in WO2004046379 (samples 1A-5A) and the primer pair mixture of the present invention (samples 1B-5B).
Figure 2 shows an example of a hyper-variable *gyrB* gene region flanked by the conserved sequences of *Staphylococcus epidermidis.* The conserved regions, situated in the 5' and 3' ends of the sequence, are marked in bold. They act as the annealing sites of broad-range *gyrB* and *parE* primers. The hyper-variable region, in which bacterial species or higher-level taxa specific probes have been designed, is between the conserved sequences (five underlined sites represent areas in which the species-specific probes have been designed).
Figure 3 shows the difference of the hybridization efficiency between specific *Pseudomonas aeruginosa gyrB* oligonucleotide probes disclosed in WO2004046379 and the improved probe disclosed herein. The microarray contained three specific *Pseudomonas aeruginosa gyrB* oligonucleotide probes; two disclosed in WO2004046379 (circled spots; SEQ ID NOs: 27/27 and 29/29) and the improved probe. disclosed herein (squared spot; SEQ ID NO: 71) that all bound specifically to the target strand. The *Pseudomonas aeruginosa* probes disclosed in WO2004046379 had significantly fainter signal intensities compared to the probe disclosed herein. Two positive control oligonucleotides were also detected (triangle-marked spots).
Figure 4 shows the result of microarray hybridization using a sample containing *Klebsiella pneumoniae* and *Enterococcus faecalis* as causative agents. The microarray contained three specific *Klebsiella pneumoniae* probes (circled spots; SEQ ID NOs: 54 and 55, one sequence not disclosed in the present invention), four specific *Enterococcus faecalis* probes (squared spots; SEQ ID NOs: 33 and 116, two sequences not disclosed in the present invention), and two *Enterobacteriaceae* family probes (hexagon-marked spots; SEQ ID NOs: 29 and 30) that bound specifically to the target strands. Two positive control oligonucleotides were also detectable (triangle-marked spots).
Figure 5 shows the result of microarray hybridization using a negative blood culture sample. The microarray contained specific *gyrB* oligonucleotides for the following bacteria *Enterobacter cloacae, Enterococcus faecalis, Enterococcus faecium, Escherichia coli, Klebsiella oxytoca, Klebsiella pneumoniae, Listeria monocytogenes, Neisseria meningitidis, Pseudomonas aeruginosa, Staphylococcus aureus, Staphylococcus epidermidis, Streptococcus agalactiae, Streptococcus pneumoniae, Streptococcus pyogenes.* Furthermore, specific probes for *Enterobacteriaceae family* and *Staphylococcus genus* were on array. No unspecific hybridization was detected. As expected, two positive control oligonucleotides (middle of the array) and ten orientation controls (five on both sides of the array) were detected.

DETAILED DESCRIPTION OF THE INVENTION

The present invention aims to provide improved probes and primers for use in the detection and identification of infection-causing bacteria, especially sepsis-causing bacteria. Examples of bacteria that cause infections, especially sepsis, include both gram-negative bacteria, such as *Acinetobacter baumannii, Bacteroides fragilis* group, *Campylobacter jejuni, Enterobacter aerogenes, Enterobacter cloacae, Escherichia coli, Haemophilus influenzae, Klebsiella oxytoca, Klebsiella pneumoniae, Neisseria meningitidis, Proteus mirabilis, Proteus vulgaris, Pseudomonas aeruginosa, Salmonella enterica* subspecies *enterica,* and *Serratia marcescens,* and gram-positive bacteria, such as *Clostridium perfringens, Enterococcus faecalis, Enterococcus faecium, Listeria monocytogenes, Staphylococcus aureus, Staphylococcus capitis, Staphylococcus epidermidis, Staphylococcus haemolyticus, Staphylococcus hominis, Staphylococcus lugdunensis, Staphylococcus warneri, Staphylococcus xylosus, Stenotrophomonas maltophilia, Streptococcus agalactiae, Streptococcus dysgalactiae, Streptococcus pneumoniae,* and *Streptococcus pyogenes.*
The topoisomerases gyrase B (gyrB or topoisomerase II) and parE proteins (the other subunit of topoisomerase IV) are important molecules for bacteria, because they are needed in the packing of DNA. Certain DNA sequence fragments of these molecules have remained almost unchanged, i.e. conserved, during evolution. In this context, the terms "a conserved region" or "conserved regions" refer to a region or regions of topoisomerase genes or proteins, whose nucleotide sequence or corresponding amino acid sequence has remained nearly unchanged between different bacterial species. Usually these conserved regions are the most important regions for the functioning of the protein. Short DNA sequences can be found in gyrB and parE molecules where the differences between various bacterial species (including closely related bacterial species) are so great that these DNA sequences can be considered as species or higher-level taxa specific. These sequences are hyper-variable sequences, which in this context refer to DNA sequences of the topoisomerase genes which differ in the nucleotide base sequence between different bacteria to an extent affording bacterial species or higher-level taxa specificity and which are situated near the conserved sequences of the topoisomerase genes and optionally limited or marked out by the conserved sequences. The term "hyper-variable sequence" also refers to the amino acid sequence encoded by said hyper-variable DNA sequence.

The present invention provides improved broad-range primers or broad-range primer mixtures that originate from conserved regions of genes encoding topoisomerases, especially the *gyrB* and *parE* genes, and that efficiently amplify DNA of infection-causing bacteria. *GyrB* gene exists as a single copy in all bacteria, while *parE* gene exists mainly in gram-negative bacteria, also as a single copy. Both the *gyrB* and *parE* genes are amplifiable by said primers.

The general design of such primers is disclosed in international patent publication WO2004046379. The primers according to the present invention (Table 1. SEQ ID NOs: 1 and 2) were designed for the same places in the sequence as the primers disclosed in WO2004046379 but the number of degenerated regions was reduced by using inosines and the sequences of clinically relevant sepsis associated bacteria were prioritized in the primer design. The sequences were obtained from the EMBL public sequence database or, in cases where such sequences were not available in public databases, they were produced by cloning the *gyrB* or *parE* gene sequence fragments from the bacterial species or by PCR with primers designed using sequences from closely related bacterial species or partial sequences available in the databases. The number of sequences used was substantially higher than the material used in WO2004046379. The data was systematically analyzed and used in primer design. In the design, inosines were placed into end of the primers since the substitution of universal bases is less destabilizing towards the termini of oligonucleotides than towards the centre. Also, possible formation of inosine-guanine base pairs was avoided.

Furthermore, the primer pair was designed to have partly overlapping melting temperatures. The melting temperature of the broad-range primer depicted in SEQ ID NO: 1 was about 8°C to about 20°C lower than the melting temperature of the broad-range primer depicted in SEQ ID NO: 2. Such a difference in the melting temperatures allows use of a novel DNA amplification method favoring production of single-stranded DNA. The method is performed in two PCR stages having different annealing temperatures. Both the primer mixture depicted in SEQ ID NO: 1 and the primer mixture depicted in SEQ ID NO: 2 anneal and extend a target DNA at the annealing temperature of the first PCR stage, while only the primer mixture depicted in SEQ ID NO: 2 anneals and extends the target DNA at the annealing temperature of the second PCR stage. Thus, the primer mixtures according to the present invention possess additional features to their broad-range or universal characteristics allowing effective amplification of single-stranded DNA. In connection with the present invention, production of single-stranded bacterial DNA is preferred although the primers may be used for producing only double-stranded bacterial DNA.

The most successful amplification of pure bacterial DNA was achieved with one of a plurality of different primer pair mixtures tested by various laboratory tests. The *gyrB* and *parE* genes of all tested bacteria could be amplified with the primer mixture according to the present invention (Example 1). The primers thus act as universal or broad-range primers for the bacteria. When compared to the primer pair mixture disclosed in WO2004046379, the primers according to the present invention were clearly more effective and sensitive (Example 2, Figure 1). The bacterial species from culture collections (ATCC and DSM) along with clinical samples were used in the specificity and sensitivity testing (Table 2).

**Table 1. *gyrB* and *parE* broad-range primers**

| Name of primer | SEQ ID NO: | Sequence 5'->3' |
|---|---|---|
| gB3F | 1 | CGICCIGGKATGTAYATHGG |
| gB4R | 2 | RMICCWACICCRTGYAGICCICC |

In the primer sequences: I represents a base inosine; K represents base G or T; Y represents base C or T; H represents base A or C or T; R represents base A or G; M represents base A or C, and W represents base A or T. The present invention thus concerns primer mixtures, which consist of several different primer alternatives. For example, in the case of primer SEQ ID NO: 1 the mixture includes primers in which K represents G (guanine) or T (thymine).

In addition, oligonucleotide analogues, such as peptide nucleic acids (PNA), or oligonucleotides comprising modified bases can act as primers and thus, are included in the meaning of the primer. The primers may consist of nucleotide sequences depicted in respective SEQ ID NOs or they may comprise additional nucleotides. Furthermore, the primers do not need to be of exactly the same sequence as the template nucleic acid but must be sufficiently complementary to hybridize with the template and retain the capability to amplify the *gyrB* and *parE* genes. Also, various chemical compounds or groups (e.g., amino groups) or other molecules, such as labels, can be attached to the primers, or they can be entirely unmodified.

Similarly, functional fragments of the previously mentioned oligonucleotide sequences are useful as primers. In this context, the term "functional fragment" refers to truncated nucleotide sequence with or without modifications, capable of hybridizing with and initiating amplification of the target DNA.

The conserved sequences of the first part of the *gyrB* and/or *parE* genes of all studied bacterial species were identified with this mixture of primers. It amplified DNA from clinical samples, and has preserved sufficiently broad specificity, thus enabling the amplification of the *gyrB* and *parE* genes from different bacterial species causing infections, especially sepsis (Example 1). In particular, this mixture of primers can be used to amplify the *gyrB* and *parE* genes of bacteria (Table 2) that are phylogenetically far from each other even in a situation where the sample includes large amounts of foreign (non-bacterial) DNA.

**Table 2. Bacterial strains used in the *gyrB* gene alignment. These bacteria were also used in sensitivity and specificity testing of primers and probes.**

| Bacterial species | Supplier's code (type of sample) |
|---|---|
| *Acinetobacter baumannii* | DSM 30007 |
| *Bacteroides fragilis* | ATCC 25285D |
| *Campylobacter jejuni* | Bacterial culture isolate |
| *Clostridium perfringens* | Bacterial culture isolate |
| *Enterobacter aerogenes* | ATCC 49469 |
| *Enterobacter cloacae* | ATCC 13047 |
| *Enterococcus faecalis* | ATCC 19433 |
| *Enterococcus faecium* | ATCC 19434 |
| *Haemophilus influenzae* | Bacterial culture isolate |
| *Escherichia coli* | DSM 30083 |
| *Klebsiella oxytoca* | DSM 5175 |
| *Klebsiella pneumoniae* | ATCC 700721 D |
| *Listeria monocytogenes* | Bacterial culture isolate |
| *Morganella morganii* | Bacterial culture isolate |
| *Neisseria meningitidis* | ATCC 700532D |
| *Proteus mirabilis* | DSM 4479 |
| *Proteus vulgaris* | Bacterial culture isolate |
| *Pseudomonas aeruginosa* | DSM 50071 |
| *Salmonella enterica* subspecies *enterica* | Bacterial culture isolate |
| *Serratia marcescens* | Bacterial culture isolate |
| *Stenotrophomonas maltophilia* | Bacterial culture isolate |
| *Staphylococcus aureus* | ATCC 10832 |
| *Staphylococcus capitis* | ATTC 27840 |
| *Staphylococcus epidermidis* | ATCC 49134 |
| *Staphylococcus haemolyticus* | DSM 20263 |
| *Staphylococcus hominis* | ATCC 25615 |
| *Staphylococcus lugdunensis* | DSM 4805 |
| *Staphylococcus warnerii* | ATCC 17917 |
| *Staphylococcus xylosus* | ATCC 35033 |

(continued)

| Bacterial species | Supplier's code (type of sample) |
|---|---|
| *Streptococcus agalactiae* | ATCC 13813 |
| *Streptococcus anginosus* | DSM 20563 |
| *Streptococcus dysgalactiae* | ATCC BAA-338 |
| *Streptococcus pneumoniae* | DSM 20566 |
| *Streptococcus pyogenes* | DSM 20565 |
| *Yersinia pseudotuberculosis* | Bacterial culture isolate |
| ATCC = American Type Culture Collection<br>DSM = Deutsche Sammlung von Mikroorganismen und Zellkulturen | |

The present application further discloses bacterial species or higher-level taxa specific oligonucleotide probes that originate from hyper-variable regions situated between the conserved regions of genes encoding topoisomerases, especially the *gyrB* and *parE* genes, these hyper-variable regions differing in the base sequences significantly between various bacterial species. With these specific probes, multiple infection-causing bacteria can be simultaneously detected and identified. In connection with the present invention, the term "higher-level taxa" refers to bacterial genera, such as *Staphylococcus* genus and *Proteus* genus, and bacterial families, such as *Enterobacteriaceae* family.

The above-mentioned features were utilized in the design of species or higher-level taxa specific oligonucleotides for multiple bacterial strains as generally disclosed in international patent publication WO2004046379. In the design of such specific probes, a planning strategy based on alignment was used. The *gyrB* or *parE* genes of target bacterial species were aligned with the corresponding genes of the non-targeted bacteria (Example 3). The sequences were obtained from the EMBL public sequence database or, in cases where such sequences were not available in public databases, they were produced by cloning the *gyrB* or *parE* gene sequence fragments from the bacterial species, or they were produced by PCR with primers designed by using sequences from closely related bacterial species or partial sequences available in the databases. An example of the hyper-variable *gyrB* gene region of *Staphylococcus epidermidis,* flanked by the conserved gene regions, is presented in Figure 2.

The specificity of the oligonucleotide probes was tested in laboratory conditions both with pure DNA samples isolated from various bacterial species and with clinical patient samples (Examples 4-5; Table 2). The improved oligonucleotide probes disclosed herein comprise the sequences selected from a group consisting of SEQ ID NOs: 3 to 121 and complementary sequences or functional fragments thereof. The probes may consist of the nucleotide sequences depicted in respective SEQ ID NOs or they may comprise additional nucleotides. Thus, they can be of different length, and only the desired species or higher-level taxa specificity and functionality of these oligonucleotide probes determine their suitable length in hybridization reactions. The length of the oligonucleotide probe is generally 15 to 60, preferably 15 to 40, and more preferably 20 to 30 nucleotides. Furthermore, the probes can be modified in different ways (e.g., oligonucleotide analogues, such as peptide nucleic acids (PNA), or oligonucleotides comprising modified bases can be used) as long as they retain the species or higher-level taxa specificity. Also, various chemical compounds or groups (e.g., amino groups) or other molecules, such as labels necessary for the detection, can be attached to the probes, or they can be entirely unmodified. The sequences of the preferred bacterial species or higher-level taxa specific probes and their specificities are presented in Table 3, and they have sequences identified with SEQ ID NOs: 3 to 121. Naturally, complementary sequences of these oligonucleotide sequences are equally suitable, as is obvious to a person skilled in the art.

Herein the term "complementary sequence" refers to a nucleic acid sequence which consists of complementary nucleotides to the original probe sequence. For example, the complementary sequence to C-A-T-G (where each letter stands for one of the bases in DNA) is G-T-A-C. Thus, the term "complementary sequence" refers to a completely complementary sequence which consists of as many nucleic acids as the original probe sequence.

Thus, each specific SEQ ID NO also refers to complementary sequences thereof.

Similarly, functional fragments of the above mentioned oligonucleotide sequences are useful as probes. In this context, the term "functional fragment" refers to a truncated sequence with or without modifications, capable of hybridizing with targeted bacteria and thus retaining an unchanged species or higher-level taxa specificity.

**Table 3. Species or higher-level taxa specific probes**

| SEQ ID NO | Specificity (gyrB) | Oligo sequence 5'->3' |
|---|---|---|
| 3 | Acinetobacter baumannii | GATGAAGCTTTAGCTGGCCACTG |
| 4 | Acinetobacter baumannii | AGTGTTTCAGATAATGGCC |
| 5 | Acinetobacter baumannii | CAGTGTTTCAGATAATGGCCGC |
| 6 | Acinetobacter baumannii | AATTATTGTCACGATTCACGAGG |
| | | |
| 7 | Bacteroides fragilis group | AGAAATCTGCCCTCGAAGTTGCC |
| 8 | Bacteroides fragilis group | AATCTGCCCTCGAAGTTGCC |
| 9 | Bacteroides fragilis group | GTATTCCGGTAGATTTCCACGAA |
| 10 | Bacteroides fragilis group | GAAGACAACTCTATCACCGTACA |
| 11 | Bacteroides fragilis group | GACCACATCGAAGTGACAATCA |
| 12 | Bacteroides fragilis group | GACCACATCGAAGTGACAATCAA |
| 13 | Bacteroides fragilis group | ACGAAGATAATTCAATCACCGTACA |
| 14 | Bacteroides fragilis group | GAAATCAGCATTGGAAGTAGCAAT |
| | | |
| 15 | Campylobacter jejuni | GATGTAGAAATCACTACTGAAGG |
| 16 | Campylobacter jejuni | TGTATAGTTAGTGATAATGGTCG |
| 17 | Campylobacter jejuni | ATATGCCAACTTTAACTGTTGTTT |
| 18 | Campylobacter jejuni | TTGATATGCACCCAACTGAAAAT |
| | | |
| 19 | Clostridium perfringens | GTAGTTGATGACGGAAGAGG |
| 20 | Clostridium perfringens | GAAGCATTAGCTGGATACTG |
| 21 | Clostridium perfringens | GGTAGTACTAGTTCAAGAGG |
| 22 | Clostridium perfringens | GGGAAAACCAACTGTAGAAG |
| | | |
| 23 | Enterobacter aerogenes | ACATCGTTGTTACGATCCACAGC |
| | | |
| 24 | Enterobacter aerogenes / Klebsiella pneumoniae | CCGCGGCATCCCAACCGGCATTC |
| 25 | Enterobacter aerogenes / Klebsiella *pneumoniae* | TCTCCGCGGCGGAAGTTAT |
| | | |
| 26 | Enterobacter cloacae | GATCCATGCGGACAACTCCGT |
| 27 | Enterobacter cloacae | GGTCACGATCCATGCGGACAA |
| 28 | Enterobacter cloacae | AGAGGGCGTATCTGCTGCG |
| | | |
| 29 | Enterobacteriaceae family | GGATGACGGCACCGGTCT |
| 30 | Enterobacteriaceae family | CTATCGACGAAGCGCTCGC |
| 31 | Enterobacteriaceae family | TATCGACGAAGCCCTCGCG |
| 32 | Enterobacteriaceae family | ATCGACGAAGCCCTCGCGGG |

(continued)

| SEQ ID NO | Specificity (gyrB) | Oligo sequence 5'->3' |
|---|---|---|
| | | |
| 33 | Enterococcus faecalis | AGCTGTGGAAACAGTGTTTACAG |
| | | |
| 34 | Enterococcus faecium | TATCACCGTTATTGACGACGGTC |
| 35 | Enterococcus faecium | TATCCAGGCAAAAACTGGACGTC |
| 36 | Enterococcus faecium | GGATACCTGTAGATATCCAGGCA |
| 37 | Enterococcus faecium | TCGATCGATGAAGTATTAGCA |
| 38 | Enterococcus faecium | ACTAGTTCTGAGGGTCTGCATCA |
| | | |
| 39 | Escherichia coli | AAATTATCGTCACCATTCACGCC |
| 40 | Escherichia coli | GGCGCGGCATTCCGACCGGTA |
| 41 | Escherichia coli | CGCCGATAACTCTGTCTCTGTA |
| 42 | Escherichia coli | TATCGGCGGCGGAAGTGAT |
| 43 | Escherichia coli | ACGGGCGCGGCATTCCGAC |
| 44 | Escherichia coli | AAGAGGGCGTATCGGCGG |
| | | |
| 45 | Haemophilus influenzae | TCTGTATCGGTGCAAGATGATGG |
| 46 | Haemophilus influenzae | GGCCATTGTTCCGATATTATCG |
| 47 | Haemophilus influenzae | GTTCCGATATTATCGTGACAA |
| 48 | Haemophilus influenzae | CTGTGGATATTCATCCTGAA |
| 49 | Haemophilus influenzae | ATGCCATTGATGAAGCCCTCGC |
| | | |
| 50 | Enterobacter aerogenes / Klebsiella oxytoca | TTACTGTAAAGACATCGTTGTTAC |
| 51 | Enterobacter aerogenes / Klebsiella oxytoca | ACTGTAAAGACATCGTTGTTACG |
| | | |
| 52 | Klebsiella oxytoca | GCGGTATTCCAACCGGTATTCA |
| 53 | Klebsiella oxytoca | GGCCGCGGTATTCCAACCGGT |
| | | |
| 54 | Klebsiella pneumoniae | TACTGCAAAGATATCGTTGTCA |
| 55 | Klebsiella pneumoniae | CTGCAAAGATATCGTTGTCAC |
| | | |
| 56 | Listeria monocytogenes | TACAATCGAAGCTGATAACAGCA |
| 57 | Listeria monocytogenes | ACTGTTCGTGATAACGGACGTGG |
| 58 | Listeria monocytogenes | GGTCGTCCAACAGTAGAAGT |
| 59 | Listeria monocytogenes | GCAATTGATGAAGCACTTGCT |
| | | |
| 60 | Neisseria meningitidis | AATCACGGTAACGATACACGC |
| 61 | Neisseria meningitidis | CCATTGACGAAGCACTCGCC |

(continued)

| SEQ ID NO | Specificity (gyrB) | Oligo sequence 5'->3' |
|---|---|---|
| 62 | Neisseria meningitidis | GTATTGGACAACGCCATTGA |
| 63 | Neisseria meningitidis | GGACATTGCGACAAAATCACG |
| | | |
| 64 | Proteus genus | ATTGTCACTATCCATTCAGATAACTC |
| 65 | Proteus genus | CATGGTGTAGGGGTTTCTGTT |
| | | |
| 66 | Proteus mirabilis | ACGGGTATTCATGAAGAAGAGG |
| 67 | Proteus mirabilis | GTATTCCAACGGGTATTCATGAA |
| 68 | Proteus mirabilis | ATGAAGAAGAGGGCGTTTCTG |
| | | |
| 69 | Proteus vulgaris | ACCAAGAAGAGGGTGTTTCAG |
| 70 | Proteus vulgaris | CCCAGAAGAGGGTGTCTCA |
| | | |
| 71 | Pseudomonas aeruginosa | GATATCCACAAGGAAGAAGGG |
| 72 | Pseudomonas aeruginosa | TCACTGTCCGCGACAATGGAC |
| 73 | Pseudomonas aeruginosa | GCCGGTTAYTGCAGCGAAATCA |
| | | |
| 74 | Salmonella enterica subspecies enterica | TCGTCGTGACTATTCACGCC |
| 75 | Salmonella enterica subspecies enterica | TAACGGATGATGGCCGTGGCATT |
| | | |
| 76 | Serratia marcescens | TTCAGCCGCAGAGGTCATCA |
| 77 | Serratia marcescens | ATTCAGGTCACCATCCATGCC |
| 78 | Serratia marcescens | TCAGGTCACCATCCATGCCG |
| 79 | Serratia marcescens | CGACAACTCGGTATCGGTG |
| | | |
| 80 | Staphylococcus aureus | AATAGTATCGATGAAGCATTAGCTG |
| 81 | Staphylococcus aureus | AACGGACGTGGTATCCCAGT |
| 82 | Staphylococcus aureus | GGACGTGGTATCCCAGTTGATAT |
| 83 | Staphylococcus aureus | TCCAGCTGTCGAAGTTATTTTAA |
| | | |
| 84 | Staphylococcus epidermidis | TAGTCATATTGAAGTTRTAATTGAG |
| 85 | Staphylococcus epidermidis | GCCCTGCTGTCGAAGTTATC |
| 86 | Staphylococcus epidermidis | CATTAGCAGGTTATGCTAGTCATA |
| 87 | Staphylococcus epidermidis | ACGCCCTGCTGTCGAAGTTA |
| 88 | Staphylococcus epidermidis | AAAGATGGGACGCCCTGCTGTCGAA |
| | | |
| 89 | Staphylococcus genus | GGCATTCCTGTTGATATTCAAGA |
| 90 | Staphylococcus genus | TCAACTTCAGAAAAAGGTTTACA |

(continued)

| SEQ ID NO | Specificity (gyrB) | Oligo sequence 5'->3' |
|---|---|---|
| 91 | Staphylococcus genus | TCAACTTCAGAAAGAGGTTTGCA |
| 92 | Staphylococcus genus | GTTGATAATAGTATTGACGAAGC |
| 93 | Staphylococcus genus | CGCCCAGCAGTTGAAGTTATCT |
| 94 | Staphylococcus genus | CCAGCAGTTGAAGTTATCTTAAC |
| 95 | Staphylococcus genus | CCAGCAGTTGAGGTTATTTTAA |
| 96 | Staphylococcus genus | GGGGCGCCCAGCAGTTGAA |
| | | |
| 97 | Stenotrophomonas maltophilia | TCGGTGTCCGACAACGG |
| 98 | Stenotrophomonas maltophilia | GGCAAGCACGAGCAGATGAG |
| 99 | Stenotrophomonas maltophilia | GCCGAAGTGGTGATGACGGT |
| | | |
| 100 | Streptococcus agalactiae | TTACATTGAACCAGATAACTCTA |
| 101 | Streptococcus agalactiae | TGGAAGACCAGCTGTAGAGACAG |
| 102 | Streptococcus agalactiae | CCTTAGCTGGATTTGCAGGACA |
| 103 | Streptococcus agalactiae | ATTCCGGTAGATATCCAAGAAAA |
| | | |
| 104 | Streptococcus dysgalactiae | ATCGTCGACAATTCTATTGACGA |
| 105 | Streptococcus dysgalactiae | ATTAGCCGGATTTGCTACTCAC |
| 106 | Streptococcus dysgalactiae | AGGCAGATAATTCAATCACAGT |
| 107 | Streptococcus dysgalactiae | CTACTCACATCCAGGTGTTTAT |
| 108 | Streptococcus dysgalactiae | ATCCAGGTGTTTATCGAGGC |
| | | |
| 109 | Streptococcus pneumoniae | TCCTGCTGTTGAGACCGTCTT |
| 110 | Streptococcus pneumoniae | TCAACCTCAAAAGAAGGTCTTCAC |
| 111 | Streptococcus pneumoniae | AGTTTTTATTGAGCCAGATGAT |
| | | |
| 112 | Streptococcus pyogenes | GTCCCGCCGTTGAAACAGTT |
| 113 | Streptococcus pyogenes | TTTTTACAGTCTTACACGCAGGT |
| 114 | Streptococcus pyogenes | GCAGGTTTTGCCTCTCATATTAAAGTCTT |
| 115 | Streptococcus pyogenes | TTACAGTCTTACACGCAGGTGG |
| | | |
| **SEQ ID NO** | **Specificity (parE)** | **Oligo sequence 5'->3'** |
| 116 | Enterococcus faecalis | CTGGTATCCCAACAGTAGAAGTA |
| 117 | Enterococcus faecium | TTTGGTCGAGGAGGGTACAAAAC |
| 118 | Staphylococcus aureus | GTAAACCGACAGTCGAAGTTATC |
| 119 | Staphylococcus aureus | CGTGGTATGCCAACAGGTAT |
| 120 | Staphylococcus aureus | GTATTTCTATAGAAGATAATGGAC |

(continued)

| SEQ ID NO | Specificity (parE) | Oligo sequence 5'->3' |
|---|---|---|
| 121 | Staphylococcus epidermidis | TGGTTATGGTGATGCGATTACAGT |
| In the SEQ ID NO: 84, R represents base A or G. | | |

Furthermore, the present invention provides a method of detecting and identifying infection-causing bacteria in a sample to be analyzed. The term "sample" as used herein encompasses a variety of sample types including bacterial and cell cultures, food samples, soil samples, biological fluids, and clini-cal specimens, such as a tissue sample, a secretion sample, such as sputum, or a brush sample, a blood sample, or another suitable sample, obtained from a patient suspected of suffering from an infectious disease. Especially, the sample to be analyzed is a positive blood culture sample suitable for clinical diagnostic applications.

In one preferred embodiment of the method according to the present invention, bacterial identification is performed using the DNA microarray technology. In this context, a DNA microarray or a DNA chip refers to a small substrate on which known nucleic acid sequences have been attached. If the nucleic acid fragments attached to the microarray are shorter than 100 base pairs (generally about 20 to about 60 base pairs), the microarray is called an oligonucleotide array.

Cloning for obtaining bacterial sequences can be performed with any conventionally known method, for example by using commercially available cloning kits (e.g., Qiagen PCR Cloning Kit, or Invitrogen TOPO TA Cloning® Kit). Sequencing of the cloning products can be performed with any sequencer suitable for this purpose (e.g., Applied Biosystems model 373A, 377 or 3100, or Bio-Rad Sequi-Gen® GT), or the products can be sequenced manually. The sequences obtained by cloning, PCR, or bioinformatic means can be analyzed manually or with sequence analysis programs designed for this purpose (e.g., Applied Biosystems Sequencer, or Invitrogen Vector NTI Advance®).

DNA may be isolated from a sample to be analyzed by any conventional method, such as commercial DNA isolation kits (e.g., Roche High Pure PCR Template Preparation Kit, BD Biosciences Clontech NucleoSpin®, or Qiagen QIAamp® DNA Mini kit) or by traditional extraction with phenolchloroform or equivalent organic solvents, either manually or with special devices that are suitable for performing DNA isolation. Automated sample preparation platforms for the isolation of DNA (e.g., bioMérieux NucliSENS® easyMAG™) are preferably used because of their general availability, rapidity, and repeatability. The DNA can also be obtained directly from the sample to be analyzed without separate isolation.

In the method of the present invention, reagents used in the DNA amplification can be any reagents that are conventionally used for the amplification of DNA, and are well known to the persons skilled in the art. Suitable and cost-effective reagents, which are commercially available, include different types of DNA polymerases and buffers therefore (e.g., AmpliTaqGOLD™, Amplitaq® LD, DyNAzyme™, TaqPlus® Precision, or HotStartTaq®), nucleotides or pre-prepared mixtures of nucleotides (e.g., Sigma, Applied Biosystems, or Amersham Biosystems), and$MgCl_2$ (whereby a product from the manufacturer of the DNA polymerase is generally used).

In the method according to the present invention, a number of known labeling methods can be used in order to produce a labeled target strand, for instance fluorescent labels (e.g., Cy5, Cy3, Cy2, TexasRed, FITC, Alexa 488, TMR, FluorX, ROX, TET, or HEX), radioactive labels (e.g., $^{32}P$, $^{33}P$, or $^{33}S$), chemiluminescent labels (e.g., HiLight Single-Color Kit), and colorimetric detection (e.g., biotin-streptavidin-enzyme conjugate). The invention also comprises the applications in which no label is needed, such as those in which the detection of nucleic acids is based on electric impulses (e.g., Motorola eSensor™).

The equipment used for amplification can be any suitable device (e.g., Biometra® T1 Thermocycler, Applied Biosystems GenAmp® PCR system 2700, or Eppendorf Mastercycler®). Practically all devices and equipment suitable for DNA amplification can be used, and amplification can also be performed manually by transferring reaction tubes from one temperature to another. In addition, amplification can be performed directly on a biochip containing specific wells for the PCR reaction, and specific hybridization area, such as a microarray, whereto the probes and control oligonucleotides can be attached.

Purification of the PCR product can be performed by any commercial method (e.g., Roche High Pure PCR Product Purification Kit, Amersham Biosciences MicroSpin™ S-400 or S-300 HR Columns, or Qiagen QIAquick® PCR-Purification-Kit) or using extraction with an organic solvent. The amplification product can also be used for the hybridization reaction as such without any purification or extraction steps.

In order to form a single-stranded target strand, any suitable method can be used including asymmetric PCR, or exonuclease treatment. The invention also comprises applications in which a double-stranded PCR product can be used in the hybridization reaction e.g. after a denaturation step.

In the hybridization reaction, each single-stranded probe hybridizes with its single-stranded target strand creating a double-stranded structure. In some embodiments the probe and the target strand hybridizes under normal hybridization conditions. A person skilled in the art is able to determine suitable hybridization conditions. The hybridization may be carried out at various hybridization temperatures (generally between 40°C and 70°C), and the time needed to perform the hybridization may vary, depending on the application, from a few minutes to a few days.

When hybridization takes place on a solid support, the probes used in the hybridization can be attached onto the surface of the solid support by covalent or non-covalent binding. Alternatively, other chemical, electrochemical or equivalent attachment methods can be used. A linker or spacer can be attached to the probe. The substrate or support onto which the probes are attached can be manufactured from glass, plastic, polymers, metal, nylon, nitrocellulose, polyacrylamide, silicon, silicon oxide, silica, or a combination thereof, and the size of the substrate can vary from a couple of millimeters to a few centimeters. The surface of the substrate can be coated with aminosilane or other appropriate surface treatment, such as epoxysilane, or alternatively a substrate that does not require any separate surface treatment can be used. Preferred substrates for the oligonucleotide probes are glass and silicon.

The synthesized probes can be printed onto the surface of the microarray support with any commercially available arrayer that is suitable for this purpose (e.g., Qarray-mini arraying system, Lucidea Array Spotter, OmniGrid® arrayer), or they can be pipetted manually onto the surface. Alternatively, the probes can be synthesized directly onto the surface by an appropriate *in situ* synthesis method, such as photolithography or ink-jet technology.

According to the invention, specific probes can be used for identification of infection-causing pathogens, especially bacteria that cause sepsis, in any suitable method, by which hybridization under normal conditions can be demonstrated for instance Southern blot, Northern blot, colony hybridization, fluorescence in situ hybridization (FISH), sequencing, and RT-PCR methods. These methods are well known to the persons skilled in the art and they can be performed both in a solution and on a solid support that binds DNA, such as on a nitrocellulose or nylon membrane or on a glass or silicon based surface.

The hybridization mixture used in hybridization may have a composition different from that presented below in the working Examples, for example, the salt composition and/or the concentration may vary (e.g., 2-4xSSC, or SSPE), or commercially available hybridization solutions can be used (e.g., Sigma ArrayHyb™). In addition, denaturing or stabilizing additives (e.g., formamide, or dimethyl sulfoxide (DMSO)) or substances that decrease nonspecific binding (e.g., bovine serum albumin (BSA), or salmon sperm DNA (ssDNA)) can be used in the hybridization mixture. Hybridization can be carried out at various hybridization temperatures (generally between 40°C and 70°C), and the time needed to perform hybridization can vary, depending on the application, from a few minutes to a few days. Instead of a water bath, hybridization can be carried out for example in an incubator or in a special hybridization device (e.g., GeneTAC HybStation, or Lucidea Slidepro Hybridizer). The post-hybridization washing steps can vary in their duration, volume, temperature, and in the composition of the washing solution, and can therefore differ from the exemplified method. The washing of the microarray can also be performed in a separate device. In some cases, washing is not necessary because the microarray can be analyzed immediately after hybridization. In a preferred method, hybridization is performed in a special hybridization device or incubator at 53°C to 58°C for about 10 to 30 minutes.

The microarray can be analyzed by any equipment or reader applicable to this purpose (e.g., GeneTAC UC4, GenePix® Personal 4100A, Agilent DNA Microarray Scanner, or a reader which consists of a light source and a camera). If the target strand is fluorescently labeled, the analysis can also be performed for example by a fluorescent microscope. If a radioactive label has been used, the array or membrane can be analyzed by autoradiography. If hybridization has been carried out on the surface of an electronic microarray and the analysis is thus based on electronic detection, the microarray can be analyzed by special equipment designed for this purpose.

The method of the present invention does not suffer from the problems of the prior art. An amplification step incorporated in the method of the present invention provides high sensitivity. The *gyrB* and *parE* genes from phylogenetically different bacterial species is amplified efficiently with the improved broad-range primers of the invention regardless of the bacterial species being gram-negative or gram-positive (Table 2). Furthermore, the PCR product is short (about 300 base pairs) improving the effectiveness of the amplification reaction.

The improved bacterial species or higher-level taxa specific probes have been designed to hybridize with the *gyrB* or *parE* genes which are considerably more variable than for example the 16S rRNA gene region that has previously been used in bacterial diagnostics. Although bacterial species of normal flora are also amplified efficiently with broad-range primers used in the present method, non-targeted identifications do not occur, because the probes are bacterial species or higher-level taxa specific and identify those bacteria for which they have been designed. All specific oligonucleotide probes were cross-tested with various bacterial species, including many bacterial species that belong to normal flora and closely related clinically relevant bacteria, to optimize the probe design. Thus, the method of the present invention is considerably more sensitive and specific than the previously described methods of similar type.

The present invention further provides a kit for detecting and identifying infection-causing bacteria, especially those causing sepsis. The kit comprises at least the DNA primer mixture of the invention as defined above in detail, any suitable and desirable mixture of improved bacterial species or higher-level taxa specific oligonucleotide probe sequences of the invention as defined above in detail, optionally attached to a solid support, optionally at least one suitable positive and/or negative control probe sequence, and optionally reagents required in the amplification, purification, hybridization, washing, and/or detection steps as discussed in detail above. A preferred kit of the invention contains an improved DNA primer mixture comprising sequences SEQ ID NOs: 1 and 2, a chip containing at least one oligonucleotide probe sequence selected from a group consisting of SEQ ID NOs: 3 to 121 attached thereto, optionally at least one positive and/or negative control, and optionally reagents required for performing the method of the invention.

A combination of oligonucleotide probes suitable for use in the present invention comprises at least two oligonucleotide probe sequences selected from a group consisting of SEQ ID NOs: 3 to 121 and complementary sequences thereof. In one embodiment, the combination may also comprise probes recognizing a single or multiple desired bacteria selected from a group consisting of *Acinetobacter baumannii, Bacteroides fragilis, Campylobacter jejuni, Clostridium perfringens, Enterobacter aerogenes, Enterobacter cloacae, Enterococcus faecalis, Enterococcus faecium, Escherichia coli, Haemophilus influenzae, Klebsiella oxytoca, Klebsiella pneumoniae, Listeria monocytogenes, Neisseria meningitidis, Proteus mirabilis, Proteus vulgaris, Pseudomonas aeruginosa, Salmonella enterica* subspecies *enterica, Serratia marcescens, Staphylococcus aureus, Staphylococcus epidermidis, Streptococcus agalactiae, Streptococcus dysgalactiae, Streptococcus pneumoniae, Streptococcus pyogenes, Stenotrophomonas maltophilia, Proteus* genus, *Staphylococcus* genus, and *Enterobacteriaceae* family.

In the following, the present invention is more precisely illustrated with the Examples. When describing the method, references have been made to different equipment, materials, temperatures, chemicals, or equivalents used in this application. These can naturally be varied in a suitable way in different applications of the invention. Therefore, the present invention and its embodiments are not limited to the Examples described below.

**Example 1. Amplification of DNA by PCR using broad-range primers**

DNA from bacterial culture isolates or clinical patient samples was amplified using the method described below.

DNA was isolated from the analyzed sample using a nucleic acid extraction robot (Nuclisens® easyMAG™, bioMérieux, France). After DNA isolation, the desired target was amplified using PCR. First, a reaction solution was prepared. The PCR reaction mixture contained 1 μM of gB3F primer mixture (SEQ ID NO: 1; ordered from Thermo Electron, USA), 1 μM of Cy5-labeled gB4R primer mixture (SEQ ID NO: 2; ordered from Thermo Electron, USA), 1xHot Start Taq® PCR buffer (Qiagen, Germany), in which $MgCl_2$ was added so that the final concentration was 2.0 mM, 300 μM of each of dATP, dGTP, dCTP, and dTTP (Finnzymes, Finland), 1.5 g/l BSA (EuroClone, Italy), 0.125 U/μl Hot Start Taq® DNA polymerase (Qiagen, Germany), and 1.5 μl isolated DNA in a total volume of 15 μl.

The PCR was performed using a thermal cycler (Mastercycler®, Eppendorf, Germany). The following PCR program was used: a 15 min denaturation step at 95°C, 36 cycles of 20 sec at 95°C, 35 sec at 52°C, 20 sec at 72°C, after which 10 cycles of 10 sec at 95°C, 30 sec at 67°C, and finally 5 cycles of 10 sec at 95°C and 30 sec at 69°C. After the PCR, the success of amplification of dsDNA and ssDNA was verified by gel electrophoresis using a 2% agarose gel containing SYBR® Green II (Invitrogen, USA).

**Example 2. PCR reaction using two different primer pair mixtures**

The properties of the primers of the present invention were studied with respect to ones disclosed in WO2004046379, which also amplify *gyrB* gene.

A set of five DNA samples extracted from culture isolates of *Staphylococcus epidermidis* was amplified using the reaction mixture described in Example 1. Two parallel reactions using the two different primer mixtures were prepared. In addition of using the primers of the present invention (SEQ ID NOs: 1 to 2), reactions using primers disclosed in WO2004046379 (SEQ ID NOs: 76 to 77) were prepared. The following PCR program was used: a 15 min denaturation step at 95°C, 36 cycles of 10 sec at 96°C, 35 sec at 52°C, and 10 sec at 72°C, after which 5 cycles of 5 sec at 96°C, 30 sec at 65°C, and finally 5 cycles of 5 sec at 96°C and 30 sec at 68°C. The amplification products were analyzed by agarose gel electrophoresis using ethidium bromide.

The results are presented in Figure 1. The primer pair mixture of the present invention produced a strong band from all the samples (1 B-5B). In contrast, amplification performed using the primers disclosed in WO2004046379 produced a strong band from three samples (1A-3A) and a very faint band from two samples (4A-5A). Thus, the primers of the present invention clearly improved the amplification reaction efficiency by providing product from all tested *S. epidermidis* strains.

**Example 3. Design of species or higher-level taxa specific probes**

In the design of bacterial species or higher-level taxa specific oligonucleotides, i.e. probes, a planning strategy based on alignment was used. The *gyrB* and *parE* genes of target bacterial species were aligned with correspondent genes of a few closely related bacterial species. For example, the *gyrB* gene of *Streptococcus pneumoniae* was aligned with *gyrB* genes of *Streptococcus pyogenes, Streptococcus mitis, Streptococcus oralis, Staphylococcus aureus,* and *Fusobacterium necrophorum. S. oralis* and *S. mitis* are closely related to *S. pneumoniae.* Therefore, oligonucleotides designed for *S. pneumoniae* must not react with these normal flora bacteria. Sequences were obtained from the EMBL public sequence database, or they were produced by cloning, or by PCR with primers designed by using sequences from closely related bacterial species or partial sequences available in the databases.

Alignment of the sequences was performed with the BioEdit program using the ClustalW alignment algorithm. The consensus sequence of the alignments was calculated and the suitably conserved regions were identified manually. These regions refer to sequence fragments that are conserved in the genes of the target bacterial species and that are not found at least entirely from the genes of the non-targeted bacteria. Oligonucleotide sequences with the suitable length (15 to 40 nucleotides) were selected from these areas for comparison analyses. The selected oligonucleotide sequences were compared to the EMBL prokaryotic sequence database using the FastA algorithm. The oligonucleotide sequences having at least two mismatches when compared to *gyrB* or *parE* sequences of non-targeted bacteria were chosen for further analyses. Theoretical melting temperature ($T_m$) was determined for oligonucleotides and the formation of secondary structures was studied. $T_m$ (°C) was calculated by the equation

$$81.5 + 16.6 \log [Na]+0.41(\%GC) - 0.61 (\%for) 500/N,$$

in which Na is the concentration of monovalent cations (50 mM in calculations), %GC is the proportion of guanine and cytosine, %for is the concentration of formamide (0% in calculations), and N is the length of the oligonucleotide. The formation of secondary structures was studied using a program provided by Sigma-Genosys. The oligonucleotides without strong secondary structures and whose $T_m$ temperature was at least 45°C were chosen for experimental specificity testing.

Oligonucleotide probes were synthesized and simultaneously modified from the 5' terminus ($NH_2$-modified oligos) (Thermo Electron, USA). The specificity of the probes was tested in the laboratory on microarrays using DNA isolated from different bacterial species (Table 2) and from patient samples. PCR for isolated DNA was performed with primer pair mixture of the present invention (SEQ ID NOs: 1 to 2). Of the tested probes, those which functioned best and had the highest specificity were selected. The sequences and specificity of the bacterial species or higher-level taxa specific probes are presented in Tables 3A and 3B.

**Example 4. Using microarrays with improved primers and probes**

The DNA of the *gyrB* gene isolated from a patient sample containing *Pseudomonas aeruginosa* as a causative agent was processed according to Example 1.

A Cy5-labeled target was hybridized with the silicon-based biochip to which the probes were attached. The hybridization reaction mixture contained around 50 to 100 ng of labeled target, 2xhybridization buffer (consisting of 1 tablet PBS pH 7.4 (Sigma, USA), 0.7 M NaCl (Fluka, Switzerland), 0.1% Tween® 20 (100%, Fluka, Switzerland), 2xDenhardt's (5x, Sigma, USA), 20 μg/ml salmon sperm DNA (dsDNA) (Amersham, United Kingdom)), hybridization control oligonucleotides, and sterile water. The volume of the reaction mixture was 25 μl. The 2xhybridization buffer was pre-warmed to 55°C before use. The hybridization reaction mixture was transferred to a hybridization area of the biochip. The hybridization area was sealed with clamps and the biochip was placed in a special hybridization device. During 10 min hybridization at 55°C, a Cy5-labeled ssDNA target bound to the complementary probe sequence immobilized on the microarray creating a labeled double-stranded structure.

After the hybridization step, the biochip was briefly washed in order to remove non-hybridized DNA. The washing step was carried out as follows: in 2xSSC solution for 1 min at 40°C, and in 0.2xSSC solution for 1 min at 40°C. After the washing, the biochip was dried. The biochip was analyzed with an optical reader, which consists of a LED light source and a CCD camera. The detection is based on a fluorescent signal released by the Cy5-labeled gene product and captured by the camera.

An example of the hybridization result is presented in Figure 3. It also illustrates the advantage provided the probes disclosed herein compared to the ones disclosed in WO2004046379. The signal intensities from probes of *Pseudomonas aeruginosa* disclosed in WO2004046379 were weak compared to the strong signal intensities from the improved probe disclosed herein. Thus, the novel design of probes clearly improved the detection of pathogens.

**Example 5. Analysis of patient samples on microarray**

DNA, extracted from a positive blood culture sample of a sepsis patient, was amplified as described in Example 1 using 2 μM primer concentrations. Both the PCR and the hybridization of the amplified DNA (described in the Example 4) were performed on the same biochip containing wells for the PCR reaction and a specific hybridization area where the probes selected from Table 3 were attached. Two bacterial species, *Klebsiella pneumoniae* and *Enterococcus faecalis,* were detected in the same sample (Figure 4). The identification was in line with the result from the blood culturing.

A negative blood culture sample was also analyzed as described above. Result revealed no signal from any of the bacterial or higher-level taxa specific probes as expected (Figure 5).

Thus, the results demonstrate simultaneous detection of several pathogens, not detectable by probes disclosed in WO2004046379, from the same sample. In addition, no unspecific hybridization from the negative sample was observed.

**Claims**

**1.** A method of detecting and identifying infection-causing bacteria in a sample, comprising the steps of

a) amplifying DNA from said sample using a mixture of DNA primers that comprises sequences which hybridize with the conserved regions of genes encoding topoisomerases, said mixture comprising SEQ ID NOs: 1 and 2,
b) contacting the amplified DNA with at least one oligonucleotide probe which hybridizes with a bacterial species or higher-level taxon specific hyper-variable region that is situated between the regions with which said DNA primers hybridize, comprising a sequence selected from a group consisting of SEQ ID NOs: 3 to 121 and complementary sequences thereof, and
c) detecting any hybridization complexes formed and identifying said infection-causing bacteria based on said detection of said hybridization complex.

**2.** The method according to claim 1, wherein said sample is a clinical sample.

**3.** The method according to claim 1 or 2, wherein said infection-causing bacteria are bacteria that cause sepsis.

**4.** The method according to any one of claims 1 to 3, wherein at least one bacterial species or higher-level taxon selected from a taxonomical group consisting of Bacillales, Bacteroidetes, Clostridia, Lactobacillales, ,Proteobacteria, *Staphylococcus* genus, *Proteus* genus and *Enterobacteri*aceae family is detected and identified.

**5.** The method according to claim 4, wherein said bacterial species is selected from a group consisting of *Acinetobacter baumannii, Bacteroides fragilis, Campylobacter jejuni, Clostridium perfringens, Enterobacter aerogenes, Enterobacter cloacae, Enterococcus faecalis, Enterococcus faecium, Escherichia coli, Haemophilus influenzae, Klebsiella oxytoca, Klebsiella pneumoniae, Listeria monocytogenes, Neisseria meningitidis, Proteus mirabilis, Proteus vulgaris, Pseudomonas aeruginosa, Salmonella enterica* subspecies *enterica, Serratia marcescens, Staphylococcus aureus, Staphylococcus epidermidis, Streptococcus agalactiae, Streptococcus dysgalactiae, Streptococcus pneumoniae, Streptococcus pyogenes,* and *Stenotrophomonas maltophilia.*

**6.** The method according to claim 5, wherein all the bacterial species depicted in claim 5 are detected and identified.

**7.** The method according to any one of claims 1 to 6, wherein in step a) the DNA amplification method is polymerase chain reaction (PCR).

**8.** The method according to any one of claims 1 to 7, wherein in step a) labeled nucleotides or labeled primers are used in the amplification of the DNA.

**9.** The method according to any one of claims 1 to 8, wherein said oligonucleotide probes are attached to a solid support.

**10.** The method according to any one of claims 1 to 98, wherein said oligonucleotide probes are in solution.

**11.** A DNA primer mixture comprising sequences hybridizing with conserved regions of genes encoding topoisomerases of infection-causing bacteria, said mixture comprising SEQ ID NOs: 1 and 2 and complementary sequences thereof.

**12.** A kit for use in the method according to claim 1, comprising

a) a DNA primer mixture according to claim 11,
b) at least one bacterial species or higher-level taxon specific oligonucleotide probe comprising a sequence selected from a group consisting of SEQ ID NOs: 3 to 121 and complementary sequences thereof, optionally attached to a solid support.

**13.** The kit according to claim 12, comprising at least one positive and/or negative control probe sequence.

**14.** The kit according to claim 12, comprising reagents required in the amplification, hybridization, purification, washing, and/or detection steps.

**Patentansprüche**

**1.** Verfahren zum Nachweisen und Identifizieren von infektionsverursachenden Bakterien in einer Probe, umfassend die Schritte

a) Amplifizieren der DNA aus der Probe unter Verwendung einer Mischung aus DNA-Primern, die Sequenzen umfassen, welche mit den konservierten Bereichen von Genen hybridisieren, die Topoisomerasen kodieren, wobei die Mischung die SEQ ID NR: 1 und 2 umfasst,
b) Inkontaktbringen der amplifizierten DNA mit mindestens einer Oligonukleotid-Sonde, die mit einem für eine Bakterienspezies oder einem Taxon höherer Ebene spezifischen hypervariablen Bereich hybridisiert, der sich zwischen den Bereichen befindet, mit denen die DNA-Primer hybridisieren, umfassend eine Sequenz, die ausgewählt ist aus einer Gruppe, die aus den SEQ ID NR: 3 bis 121 und komplementären Sequenzen davon besteht, und
c) Nachweisen von allen gebildeten Hybridisierungskomplexen und Identifizieren der infektionsverursachenden Bakterien auf der Grundlage des Nachweises des Hybridisierungskomplexes.

**2.** Verfahren nach Anspruch 1, wobei die Probe eine klinische Probe ist.

**3.** Verfahren nach Anspruch 1 oder 2, wobei die infektionsverursachenden Bakterien Bakterien sind, die eine Sepsis verursachen.

**4.** Verfahren nach einem der Ansprüche 1 bis 3, wobei mindestens eine Bakterienspezies oder ein Taxon höherer Ebene, ausgewählt aus einer taxonomischen Gruppe, die aus Bacillales, Bacteroidetes, Clostridia, Lactobacillales, Proteobacteria, *Staphylococcus*-Gattung, *Proteus*-Gattung und *Enterobacteriaceae-Familie* besteht, nachgewiesen und identifiziert wird.

**5.** Verfahren nach Anspruch 4, wobei die Bakterienspezies ausgewählt ist aus einer Gruppe, die aus *Acinetobacter baumannii, Bacteroides fragilis, Campylobacter jejuni, Clostridium perfringens, Enterobacter aerogenes, Enterobacter cloacae, Enterococcus faecalis, Enterococcus faecium, Escherichia coli, Haemophilus influenzae, Klebsiella oxytoca, Klebsiella pneumoniae, Listeria monocytogenes, Neisseria meningitidis, Proteus mirabilis, Proteus vulgaris, Pseudomonas aeruginosa, Salmonella enterica* Subspezies *enterica, Serratia marcescens, Staphylococcus aureus, Staphylococcus epidermidis, Streptococcus agalactiae, Streptococcus dysgalactiae, Streptococcus pneumoniae, Streptococcus pyogenes* und *Stenotrophomonas maltophilia* besteht.

**6.** Verfahren nach Anspruch 5, wobei alle Bakterienspezies, die in Anspruch 5 geschildert sind, nachgewiesen und identifiziert werden.

**7.** Verfahren nach einem der Ansprüche 1 bis 6, wobei in Schritt a) das Verfahren zur DNA-Amplifikation die Polymerase-Kettenreaktion (PCR) ist.

**8.** Verfahren nach einem der Ansprüche 1 bis 7, wobei in Schritt a) markierte Nukleotide oder markierte Primer bei der Amplifikation der DNA verwendet werden.

**9.** Verfahren nach einem der Ansprüche 1 bis 8, wobei die Oligonukleotid-Sonden an einem festen Trägermaterial befestigt sind.

**10.** Verfahren nach einem der Ansprüche 1 bis 8, wobei die Oligonukleotid-Sonden in Lösung sind.

**11.** DNA-Primermischung, die Sequenzen umfasst, welche mit konservierten Bereichen von Genen hybridisieren, die Topoisomerasen von infektionsverursachenden Bakterien kodieren, wobei die Mischung die SEQ ID NR: 1 und 2 und komplementäre Sequenzen davon umfasst.

**12.** Kit zur Verwendung in dem Verfahren nach Anspruch 1, umfassend

a) eine DNA-Primermischung nach Anspruch 11,
b) mindestens eine spezifische Oligonukleotid-Sonde einer Bakterienspezies oder einem Taxon höherer Ebene, die eine Sequenz umfasst, welche ausgewählt ist aus einer Gruppe, die aus den SEQ ID NR: 3 bis 121 und komplementären Sequenzen davon besteht, die gegebenenfalls an einem festen Trägermaterial befestigt ist.

**13.** Kit nach Anspruch 12, das mindestens eine positive und/oder negative Kontrollsonden-Sequenz umfasst.

**14.** Kit nach Anspruch 12, das Reagenzien umfasst, die bei dem Amplifikations-, dem Hybridisierungs-, dem Reinigungs-, dem Wasch- und/oder dem Nachweisschritt benötigt werden.

**Revendications**

**1.** Procédé de détection et d'identification de bactéries génératrices d'infections dans un échantillon, comprenant les étapes consistant à :

a) amplifier l'ADN dudit échantillon en utilisant un mélange d'amorces d'ADN qui comprend des séquences qui s'hybrident avec les régions conservées de gênes codant pour des topoisomérases, ledit mélange comprenant les SEQ ID n° 1 et 2,
b) mettre en contact l'ADN amplifié avec au moins une sonde oligonucléotidique qui s'hybride avec une espèce bactérienne ou une région hypervariable spécifique d'un taxon de niveau supérieur, qui est située entre les régions avec lesquelles lesdites amorces d'ADN s'hybrident, comprenant une séquence choisie dans un groupe constitué des SEQ ID n° 3 à 121 et de leurs séquences complémentaires, et
c) détecter tous complexes d'hybridation formés et identifier lesdites bactéries génératrices d'infections sur la base de ladite détection dudit complexe d'hybridation.

**2.** Procédé selon la revendication 1, dans lequel ledit échantillon est un échantillon clinique.

**3.** Procédé selon la revendication 1 ou la revendication 2, dans lequel lesdites bactéries génératrices d'infections sont des bactéries qui provoquent une septicémie.

**4.** Procédé selon l'une quelconque des revendications 1 à 3, dans lequel au moins une espèce bactérienne ou un taxon de niveau supérieur choisi dans un groupe taxonomique constitué des Bacillales, des Bactéroïdètes, de la Clostridia, des Lactobacillales, des Protéobactéries, du genre *Staphylococcus,* du genre *Proteus* et de la famille des *Enterobacteriaceae* est détecté(e) et identifié(e).

**5.** Procédé selon la revendication 4, dans lequel ladite espèce bactérienne est choisie dans un groupe constitué des suivantes : *Acinetobacter baumannii, Bacteroides fragilis, Campylobacter jejuni, Clostridium perfringens, Enterobacter aerogenes, Enterobacter cloacae, Enterococcus faecalis, Enterococcus faecium, Escherichia coli, Haemophilus influenzae, Klebsiella oxytoca, Klebsiella pneumoniae, Listeria monocytogenes, Neisseria meningitidis, Proteus mirabilis, Proteus vulgaris, Pseudomonas aeruginosa, Salmonella enterica* sous-espèce *enterica, Serratia marcescens, Staphylococcus aureus, Staphylococcus aureus, Staphylococcus epidermidis, Streptococcus agalactiae, Streptococcus dysgalactiae, Streptococcus pneumoniae, Streptococcus pyogenes* et *Stenotrophomonas maltophilia.*

**6.** Procédé selon la revendication 5, dans lequel toutes les espèces bactériennes décrites dans la revendication 5 sont détectées et identifiées.

**7.** Procédé selon l'une quelconque des revendications 1 à 6, dans lequel, à l'étape a), le procédé d'amplification de l'ADN est l'amplification en chaîne par polymérase (ACP).

**8.** Procédé selon l'une quelconque des revendications 1 à 7, dans lequel, à l'étape a), des nucléotides marqués ou des amorces marquées sont utilisé(e)s dans l'amplification de l'ADN.

**9.** Procédé selon l'une quelconque des revendications 1 à 8, dans lequel lesdites sondes oligonucléotidiques sont fixées à un support solide.

**10.** Procédé selon l'une quelconque des revendications 1 à 8, dans lequel lesdites sondes oligonucléotidiques sont en solution.

**11.** Mélange d'amorces d'ADN comprenant des séquences s'hybridant avec des régions conservées de gènes codant pour des topoisomérases de bactéries génératrices d'infections, ledit mélange comprenant des SEQ ID n° 1 et 2 et des séquences complémentaires de celles-ci.

**12.** Kit pour usage dans le procédé selon la revendication 1, comprenant :

a) un mélange d'amorces d'ADN selon la revendication 11,
b) au moins une espèce bactérienne ou une sonde oligonucléotidique spécifique à un taxon d'ordre supérieur comprenant une séquence choisie dans le groupe constitué des SEQ ID n° 3 à 121 et de leurs séquences complémentaires, éventuellement fixées à un support solide.

**13.** Kit selon la revendication 12, comprenant au moins une séquence de sonde témoin positive et/ou négative.

**14.** Kit selon la revendication 12, comprenant des réactifs requis dans les étapes d'amplification, d'hybridation, de purification, de lavage et/ou de détection.

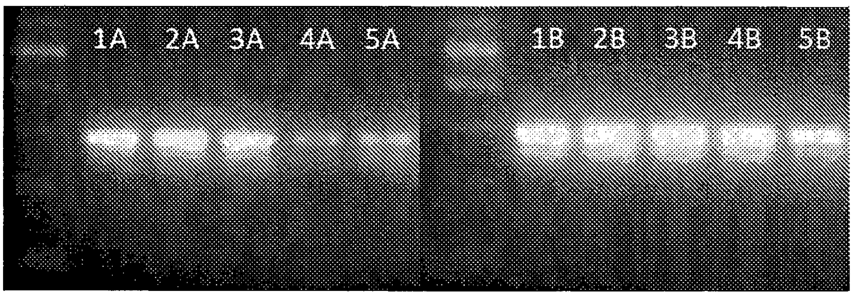


Figure 1.

5´- **AGACCRGGTA TGTATATTGG** TTCAACTTCA GAAAGAGGGT TGCACCATTT AGTATGGGAA ATTGTTGATA ATAGTATTGA

CGAGGCATTA GCAGGTTATG CTAGTCATAT TGAAGTTRTA ATTGAGAAAG ACAATTGGAT TAAAGTTACT GACAATGGCC

GTGGTATTCC TGTTGATATT CAAGAAAAGA TGGGACGCCC TGCTGTCGAA GTTATMTTAA CTGTACTTCA CGCTGGRGGT

AAATTYGGAG GTGGCGGATA CAAAGTATCT **GGCGGTCTTC ACGGTGTTGG ATC** -3´

Figure 2.

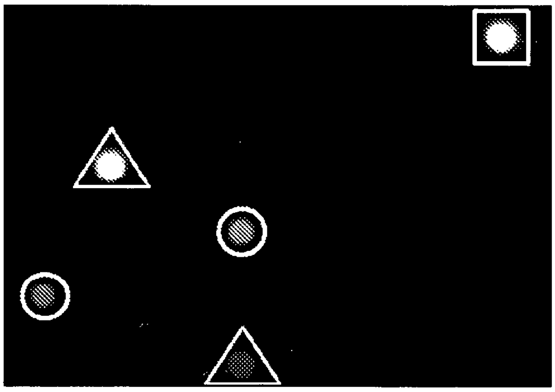

Figure 3.

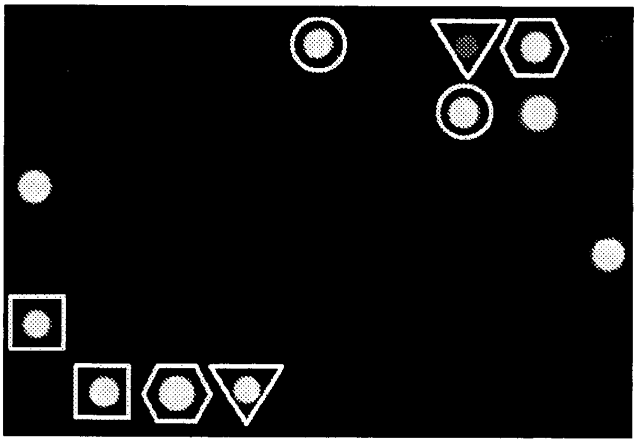

Figure 4.

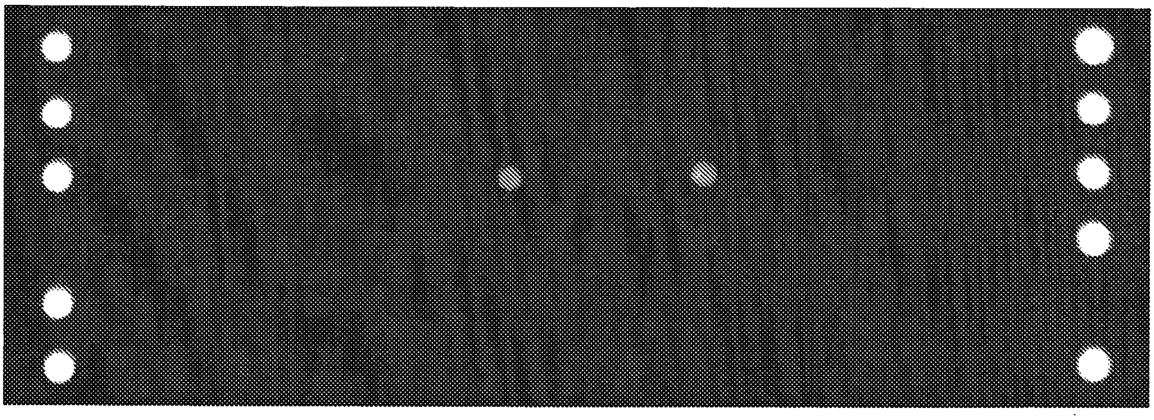

Figure 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 613502 B1
- US 6001564 A
- US 20020055101 A
- WO 2004046379 A


### Non-patent literature cited in the description


- **DAUGA.** *Int. J. Syst. Evol. Microbiol.,* 2002, vol. 52, 531-547